# EUROPEAN PATENT APPLICATION

(11) **EP 3 096 140 A1**
(43) Date of publication of application: **23.11.2016**
(21) Application number: 14879160.1
(22) Date of filing: 14.10.2014
(51) Int. Cl.: G01N 33/558

(54) **NANOMETER MIMIC ENZYME IMMUNOCHROMATOGRAPHY DETECTION METHOD**

(30) Priority: 14.01.2014 CN 201410015610
(71) Applicant: The Institute of Biophysics Chinese Academy of Sciences, Beijing 100101 (CN); Gill Biotechnology (Tianjin) Co., Ltd., Tianjin 300270 (CN)
(72) Inventor: YAN, Xiyun, Beijing 100101 (CN); DUAN, Demin, Beijing 100101 (CN); ZHANG, Dexi, Beijing 100101 (CN); YANG, Dongling, Beijing 100101 (CN); FENG, Jing, Beijing 100101 (CN); SANG, Lina, Beijing 100101 (CN)
(74) Representative: dompatent von Kreisler Selting Werner - Partnerschaft von Patent- und Rechtsanwälten mbB
(86) International application number: PCT/CN2014/088540
(87) International publication number: WO 2015/106588

(57) **Abstract**

This invention provides a nanozyme immunochromatographic detection method for detecting a substance to be tested in a liquid sample, comprising, in this order, the steps of:1) providing a detection probe, which is prepared by coupling a magnetic nanoparticle to a first molecule capable of specifically binding to the substance to be tested;2) providing a capture probe, which is an immobilized second molecule capable of specifically binding to the substance to be tested;3) bringing the liquid sample into contact with the detection probe;4) bringing the liquid sample, which has been in contact with the detection probe, into contact with the capture probe; and 5) adding a hydrogen-donor substrate and a peroxide to the capture probe subject to the step 4) so as to perform color development reaction. This invention further provides a nanozyme immunochromatographic detection apparatus for detecting a substance to be tested in a liquid sample.

## Description

### Technical Field

The present invention relates to nanomaterials and biomedical nanotechnology. The present invention relates to a magnetic nanoparticle nanozyme, and provides a method of immunochromatographic detection of biological molecules using the same.

### Background Art

Colloidal gold immunochromatography is a detection method with colloidal gold as a label, which has been developed since early 1990s, and it combines immunoaffinity technology, immunoblotting technology, and spot thin-layer chromatography technology. Since all samples undergo a continuous reaction through a relatively narrow celluloses membrane when detecting with the chromatographic tape, the substance to be tested is actually concentrated and aggregated, which improves the sensitivity of the reaction and increases the reaction rate, requiring only 3-15 min for the whole operation. The principle is as follows: a specific antibody (or antigen) is first immobilized on a certain zone of a nitrocellulose membrane; once one end of the dry nitrocellulose membrane is dipped into a sample, the sample will move forward along the membrane (chromatography) due to capillary action; and once moving to the zone immobilized with the antibody or antigen, a corresponding antigen in the sample specifically binds to the antibody, and immuno-colloidal gold as a label may visualizing the zone as a certain color so as to achieve specific immunodiagnosis. Conventional colloidal gold immunochromatographic techniques have the characteristics of easy operation, being economical, and high speed. However, due to relatively low sensitivity, its wide application in terms of biomolecular detection is severely restricted, and signal amplification is the key point for solving the low sensitivity of immunochromatographic techniques.

Magnetic nanoparticles, which have good biocompatibility, have not only the properties specific for nanomaterials such as small particle size, large specific surface area, and high coupling capacity, but also magnetic responsiveness and superparamagnetism, may be aggregated and localized under a constant magnetic field, and generate heat by absorbing electromagnetic waves under an alternating magnetic field. With these characteristics, magnetic nanoparticles are widely applied in biological fields, such as contrast agents for magnetic resonance, magnetic targeting drug carriers, separation of cells and biological molecules, biosensing and detection, tumor thermotherapy by magnetic induction, and the like.

In recent years, magnetic immunochromatographic test (MICT) has been gradually developed as a new-generation individual rapid quantitative detection technique, in which immunochromatography is performed using superparamagnetic nanoparticles in place of conventional labels (such as colloidal gold, latex particles, and the like) and finally the magnetic field strength of the magnetic particles binding to the test line is read by a magnetic signal reader so that qualitative and quantitative measurement of a sample to be tested can be performed. At present, domestic magnetic signal readers have not been in market, and only very few companies (such as MagnaBioSciences Corporation, US) possess this technology, which, however, is high in price, and thus limits the development and popularization of magnetic immunochromatography.

In recent years, our research team has studied and found that magnetic nanoparticles have intrinsic enzyme mimic activity (named nanozyme) and can replace peroxidase for performing immunodetection (Xiyun Yan et al., Nature Nanotechnology. 2007). Recently, our research team has further developed a new immunohistochemical detection reagent which integrates three functions including recognition, catalysis, and magnetism, and has found that the surface of a magnetic particle still has enzymatic activity even after being coated with protein molecules (Xiyun Yan et al., Nature Nanotechnology. 2012). This catalytic activity is similar to that of horse radish peroxidase. Magnetic nanoparticles may, in the presence of hydrogen peroxide, catalyze the substrate of horse radish peroxidase, and for example, catalyze 3,3,5,5-tetramethyl benzidine (TMB) to generate a blue product, catalyze diaminobenzidine (DAB) to generate a brown precipitate, catalyze o-phenylene diamine (OPD) to generate an orange product. Catalytic activity depends on pH value, temperature, and hydrogen peroxide concentration, and its catalytic mechanism complies with Ping-Pong mechanism. Meanwhile, it is also found that the catalytic activity of magnetic nanoparticles increases as the particle size of the particles decreases, and the smaller the particle size of the particles is, the higher the catalytic activity thereof is. When the particle size of magnetic particles comes to micrometer scale, the catalytic activity was reduced nearly to zero. Compared to horse radish peroxidase (HRP) which is a protein, the nanozyme activity of magnetic nanoparticles has more advantages: (1) proteases tend to be denatured at an extreme pH and temperature and also tend to be degraded by proteases, while magnetic nanoparticles are very stable under extreme conditions; (2) the production cost of a protease is very high, while the preparation of magnetic nanoparticles is simple and inexpensive; and (3) magnetic nanoparticles having superparamagnetism may be recovered for repeated utilization; meanwhile, based on magnetic controllability of magnetic nanoparticles, the field of application in which they are used as enzyme mimic has been extended.

### Summary of the Invention

The object of the present invention is to overcome the deficiencies of the immunochromatography techniques in the prior art, and to provide a nanozyme immunochromatographic detection method, which employs the same basic principle as that of colloidal gold test strip: an antibody A, which is specific to a certain antigen, is first coupled to a magnetic nanoparticle to prepare a magnetic particle pad; an antibody B which is also specific to the antigen is then immobilized onto a certain band on a nitrocellulose membrane to form a test line (T line); an antibody against the antibody A (secondary antibody) is also immobilized on a certain zone on the nitrocellulose membrane to form a control line (C line) parallel to T line; and finally a magnetic immunochromatographic test paper is assembled and prepared. When one end of dry nitrocellulose membrane is dipped into a sample, the sample will move forward along the membrane due to capillary action. Once moving to the magnetic particle pad, the antigen in the sample will react with the antibody A on the magnetic particle to generate an antigen-antibody A-magnetic particle complex, which continues to move by capillary action to the T line zone immobilized with the antibody B, the antigen in the sample will react with the antibody B to finally generate an antibody B-antigen-antibody A-magnetic particle complex; while magnetic particle-antibody probes without antigens bound continue to move forward and bind to the secondary antibody at the C line to form a secondary antibody-antibody A-magnetic particle complex, thus magnetic particles being aggregated at the T line and the C line. If the concentration of antigens in the sample is relatively high, there will be a large number of magnetic particles aggregating at the T line, showing the color of the magnetic particles. If the concentration of antigens in the sample is very low, there will be very few magnetic particles aggregating at the T line, not allowing the color of the magnetic particles to be shown. In this case, a peroxide and a hydrogen-donor substrate (such as TMB, DAB, and the like) are added, which results in a larger amount of precipitate via the nanozyme catalytic action of the magnetic particles so that detection signal is enhanced, which enables the detection of the antigen at a low concentration. This technique combines the peroxidase catalytic activity and magnetic separation property of magnetic nanoparticles. Brown precipitate is generated by adding a peroxide and a hydrogen-donor substrate such as o-phenylene diamine (DAB) and the like after chromatography via the nanozyme catalytic action of magnetic particles so as to enhance detection signal, improve sensitivity, visualize detection results, and eliminate the dependency on instruments. The detection of biological specimens based on this new technique is convenient and rapid, and is quite suitable for on-site use.

This invention is achieved by the following technical solutions:
a nanozyme immunochromatographic detection method, comprising: 1) employing magnetic nanoparticles having a suitable size, and preparing specific nanoparticle probes by coupling biological molecules which specifically bind to antigens to be tested to the surface of the magnetic nanoparticles so as to prepare a magnetic particle pad; 2) assembling and preparing a magnetic immunochromatographic test paper; 3) chromatographic reaction, in which the antigen to be tested, the magnetic nanoprobe, a test line antibody, and a control line antibody form a sandwich complex, and a positive sample will result in the aggregation of the magnetic particles at the T line; 4) color development reaction, in which a peroxide and a hydrogen-donor substrate (such as TMB, DAB, and the like) are added and a large amount of precipitate is generated via nanozyme catalytic action of the magnetic particles so that detection signal is enhanced; and 5) achieving the qualitative and semi-quantitative detection of target molecules based on the experimental results.

Furthermore, this invention provides a nanozyme immunochromatographic detection method, wherein said assembling and preparing a magnetic immunochromatographic test strip comprises allowing a coating membrane, a magnetic particle pad which binds to an antibody against the antigen to be tested, a sample pad, and a water-absorbent pad to sequentially adhere to a base plate in a mutually staggered form, and then assembling by covering the upper layer with a transparent plastic sealing film, wherein said coating membrane is precoated with a test line of the antigen to be tested and a control line.

Furthermore, the nanozyme immunochromatographic detection method described above is characterized in that the magnetic nanoparticle may have a shape of any one of spherical, rod-shaped, cubic, triangular, polygonal shape, and the like; it may have a particle size in a range of 10 nanometers to 500 nanometers; it may be a naked magnetic particle or a magnetic particle coated with a protein shell such as a viral envelope, a transferrin shell, or a ferritin shell; and it may be Fe₃O₄ magnetic particle, or Fe₂O₃ magnetic particle modified with bivalent iron ion reagent on the outer layer.

Furthermore, the nanozyme immunochromatographic detection method described above is characterized in that the hydrogen-donor substrate includes tetramethyl benzidine TMB, tetramethyl benzidine sulfate TMBS, o-phenylene diamine OPD, diaminobenzidine DAB, diaminobenzidine tetrahydrochloride DAB-4HCl, 5-amino salicylic acid 5-AS, o-tolidine OT, or 2,2'-azino-bis(3-ethylbenzothiazoline-6-sulfonic acid) diammonium salt (ABTS); and the peroxide includes hydrogen peroxide or urea peroxide and the like.

Furthermore, the nanozyme immunochromatographic detection method described above is characterized in that the specific biological molecule includes protein, nucleic acid, and polypeptide; the target molecule is present in solution or body fluid.

The prominent substantive features and notable progress of the technical solutions of the invention are as follows.

This invention provides a nanozyme immunochromatographic detection method to conquer the deficiencies of current colloidal gold technology which does not have the function of signal amplification and has a relatively low sensitivity, based on the latest scientific findings. The present technique combines peroxidase catalytic activity of magnetic nanoparticles with magnetic separation property of magnetic nanoparticles. A large amount of brown precipitate is generated by adding a peroxide and a hydrogen-donor substrate such as o-phenylene diamine (DAB) and the like after chromatography by using nanozyme activity of the magnetic particles so as to amplify detection signal by 10-100 times, which enables the visualization of the detection results, and eliminate the dependency on instruments. The detection of biological specimens based on this new technique is convenient and rapid and has a high sensitivity, and is quite suitable for on-site use. It is a new technique having novelty, inventiveness, and applicability.

More specifically, this invention provides the following:
1. A nanozyme immunochromatographic detection method for detecting a substance to be tested in a liquid sample, comprising the steps of:
   1) providing a detection probe, which is prepared by coupling a magnetic nanoparticle to a first molecule capable of specifically binding to the substance to be tested;
   2) providing a capture probe, which is an immobilized second molecule capable of specifically binding to the substance to be tested;
   3) bringing the liquid sample into contact with the detection probe;
   4) bringing the liquid sample which has been in contact with the detection probe, into contact with the capture probe; and
   5) adding a hydrogen-donor substrate and a peroxide to the capture probe which has been subjected to the step 4) so as to carry out a color development reaction.
2. The method according to 1, wherein the particle size of the magnetic nanoparticle is in a range of 10 nanometers to 500 nanometers.
3. The method according to 1, wherein the magnetic nanoparticle is Fe₃O₄ magnetic nanoparticle.
4. The method according to 1, wherein the substance to be tested is a protein, polypeptide, or nucleic acid.
5. The method according to 1, wherein the substance to be tested is protein, and the first molecule and the second molecule are specific antibodies, preferably monoclonal antibodies, against the protein.
6. The method according to 5, wherein the first molecule and the magnetic nanoparticle are coupled by EDC-NHS method.
7. The method according to 1, wherein the hydrogen-donor substrate includes tetramethyl benzidine (TMB), tetramethyl benzidine sulfate (TMBS), o-phenylene diamine (OPD), diaminobenzidine (DAB), diaminobenzidine tetrahydrochloride (DAB-4HCl), 5-amino salicylic acid (5-AS), o-tolidine (OT), or 2,2'-azino-bis(3-ethylbenzothiazoline-6-sulfonic acid) diammonium salt (ABTS).
8. The method according to 1, wherein the peroxide includes hydrogen peroxide and urea peroxide.
9. A nanozyme immunochromatographic detection apparatus for detecting a substance to be tested in a liquid sample, comprising the following provided on a base plate:
   a sample pad, which is used for bearing the liquid sample and filtering impurities in the sample;
   a magnetic nanoparticle pad, which comprises magnetic nanoparticles coupled to first molecules capable of specifically binding to the substance to be tested;
   a test line, which comprises second molecules capable of specifically binding to the substance to be tested; and
   an absorbent pad, which is generally made of a relatively thick filter paper or a similar water-absorbent material and is used for providing a driving force for chromatography,
      wherein the particle size of the magnetic nanoparticle is preferably in a range of 10 nanometers to 500 nanometers,
      wherein the magnetic nanoparticle is preferably Fe₃O₄ magnetic nanoparticle,
      wherein the substance to be tested is preferably protein, polypeptide, or nucleic acid, and more preferably, the substance to be tested is protein, while the first molecule and the second molecule are specific antibodies, preferably monoclonal antibodies, against the protein,
      wherein the first molecule and the magnetic nanoparticle are preferably coupled by EDC-NHS method,
      wherein the hydrogen-donor substrate preferably includes tetramethyl benzidine (TMB), tetramethyl benzidine sulfate (TMBS), o-phenylene diamine (OPD), diaminobenzidine (DAB), diaminobenzidine tetrahydrochloride (DAB-4HCl), 5-amino salicylic acid (5-AS), o-tolidine (OT), or 2,2'-azino-bis(3-ethylbenzothiazoline-6-sulfonic acid) diammonium salt (ABTS), and
      wherein the peroxide preferably includes hydrogen peroxide and urea peroxide. 10. The detection apparatus according to 9, further comprising, behind the test line, a control line at which third molecules capable of specifically binding to the first molecule are immobilized.

### Brief Description of the Drawings

Hereafter, the technical solutions of the invention will be further illustrated with respect to the accompanying drawings:

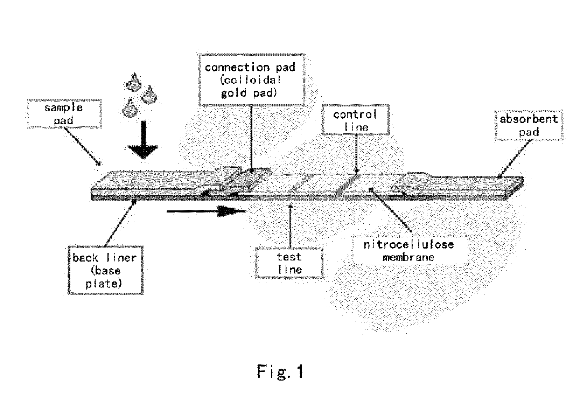

FIG.1: A schematic view of colloidal gold immunochromatography;
FIG.2: A schematic view of an embodiment of the nanozyme immunochromatography according to the invention;
FIG.3: Screening abrin monoclonal antibodies with high affinity by enzyme-linked immunoadsorbent assay (ELISA) method;
FIG.4: Identification of subgroups of the abrin monoclonal antibodies;
FIG.5: Identification of the epitope on abrin recognized by the monoclonal antibodies by Western blotting method;
FIG.6: Screening pairing antibodies of abrin by double-antibody sandwich enzyme-linked immunoadsorbent assay (ELISA) method;
FIG.7: Preparation of magnetic nanoparticles;
FIG.8: Preparation of magnetic particle antibody probes and Dot blot examination;
FIG.9: Comparison of the sensitivity for detecting abrin by using the magnetic particle nanozyme immunochromatographic method and by using colloidal gold immunochromatographic method; and
FIG.10: Comparison of sensitivity for detecting influenza virus by using nanozyme immunochromatographic method and by using the conventional colloidal gold method.

### Description of Embodiments

Hereafter, this invention will be described with respect to specific Examples, and it is to be understood that the scope of the invention is not limited to the specific Examples.

### Example 1: Detection of abrin by nanozyme immunochromatographic detection method

### 1) Preparation of hybridoma cells

Abrin, which is a component in seeds of *Abrus precatorius* (a Fabaceae plant), is one of the most toxic plant toxins ever found, and is considerably toxic to human, animals, and insects. It is lethal to chew even only one *Abrus precatorius* seed. Here, the sensitivity and applicability of the present nano-enzyme mimic immunochromatographic detection method is illustrated by taking the detection of abrin (both abrin and ricin mentioned hereafter were provided by Academy of Military Medical Sciences, China) as an example. Monoclonal antibodies against abrin, Abrin-1, Abrin-2, Abrin-3, and Abrin-4 used in the experiments were derived from the hybridoma cells secreting monoclonal antibodies Abrin-1, Abrin-2, Abrin-3, and Abrin-4, respectively. The method for preparing the monoclonal antibodies is known in the art, and can be seen, for example, in Kohler and Milstein, Nature 256:495, 1975; Yeh et al.,Proc.Natl.Acad.Sci. USA, 1979; and Yeh et al., Int.J.Cancer,1982*,* and specifically is as follows: crude toxin obtained from *Abrus precatorius* seeds by ammonium sulfate precipitation method was subjected to processes such as molecular sieve column chromatography, ion exchange column chromatography, and freeze-drying to provide a highly pure abrin which, after being inactivated with formaldehyde, was used as an immunogen (whole toxin) to inoculate BALB/C mice, 20 µg of the protein/mouse subcutaneously injected every two weeks for three times. The immunization was boosted once before employing spleen cells. On Day 3 after boosting immunization, the spleen was taken, and spleen cells were suspended in RPMI culture medium. The spleen cells were fused with mouse myeloma SP2/0-Ag14 cells in the presence of polyethylene glycol (PEG), and the hybridomas were screened by using HAT selective culture medium (a culture medium containing hypoxantin, aminopterin, and thymidin) to provide hybridoma cells. Antibodies having a high affinity to native abrin were screened by ELISA method to obtain four strains of antibodies, designated as Abrin-1, Abrin-2, Abrin-3, and Abrin-4, respectively, and also the hybridoma cells secreting these antibodies, Abrin-1, Abrin-2, Abrin-3, and Abrin-4.

### 2) Identification of the affinity of the monoclonal antibodies Abrin-1, Abrin-2, Abrin-3, and Abrin-4 for abrin by ELISA method

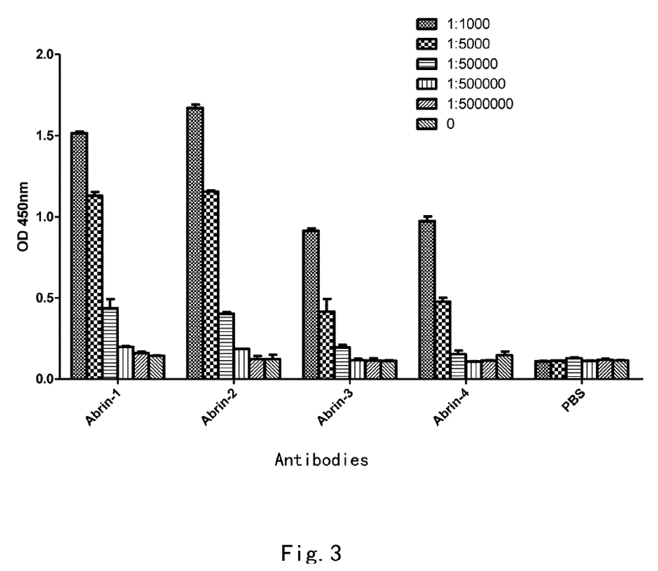

First, a 96-well ELISA plate was coated with 50 µl of 2 µg/ml abrin protein overnight, washed three times with PBST, and blocked with 5% BSA-PBS for 1 h. The culture supernatants of the hybridoma cells of the monoclonal antibodies were added, and incubated at 37°C for 1 h, and washed three times with PBST. Then, an HRP-labeled goat anti-mouse antibody was added, and incubated for 1 h, and washed three times with PBST. A chromogenic substrate TMB (200 ng/ml of TMB, 0.03% of H₂O₂, pH 4.5) was added at 50 µl/well for color development. The reaction was performed at 37°C for 15min. Then, 2M sulfuric acid solution was added at 50 µl/well to terminate the reaction. The results were obtained by using a microplate reader at 450 nm. From the results of ELISA, it can be seen that the affinity of Abrin-3 and Abrin-4 may reach 1:5000, while the affinity of Abrin-1 and Abrin-2 are up to 1:50000 (as shown in FIG. 3).

### 3) Purification of the monoclonal antibodies by affinity chromatography

The hybridoma cells, Abrin-1, Abrin-2, Abrin-3, and Abrin-4, were cultured and proliferated, and were prepared into cell suspension. 6-week old BALB/C mice were intraperitoneally injected with pristane (Sigma-Aldrich) at 0.5 ml/mouse. After about ten days, ascites were collected and centrifuged to obtain supernatant. The monoclonal antibodies were purified from the ascites by protein G affinity chromatography (Roche). The purified monoclonal antibodies were sterilized by filtration, and refrigerated or frozen for storage.

### 4) Identification of the subgroups of the monoclonal antibodies

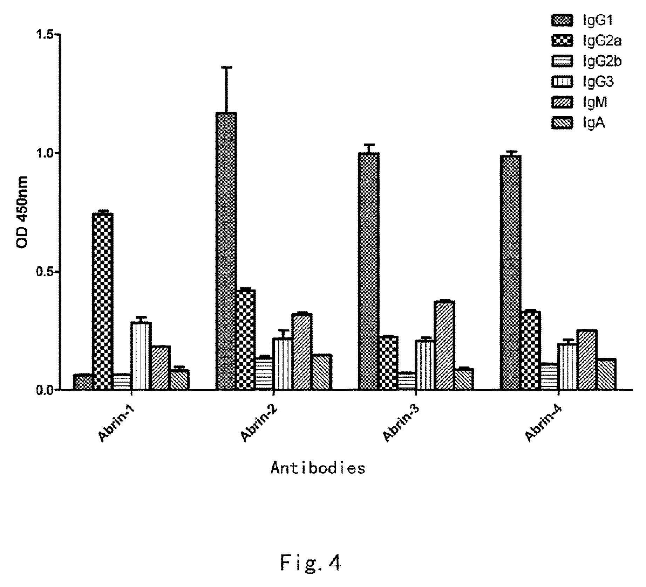

The identification was performed by using a mouse antibody subgroup identification kit (BD Pharmingen) according to the manufacturer's instructions. As a result, the antibody Abrin-1 was identified as belonging to subgroup IgG2a and, the antibodies Abrin-2, Abrin-3, and Abrin-4 belong to subgroup IgG1 (as shown in FIG.4).

### 5) Identification of the epitope on abrin recognized by the antibodies by Western blotting

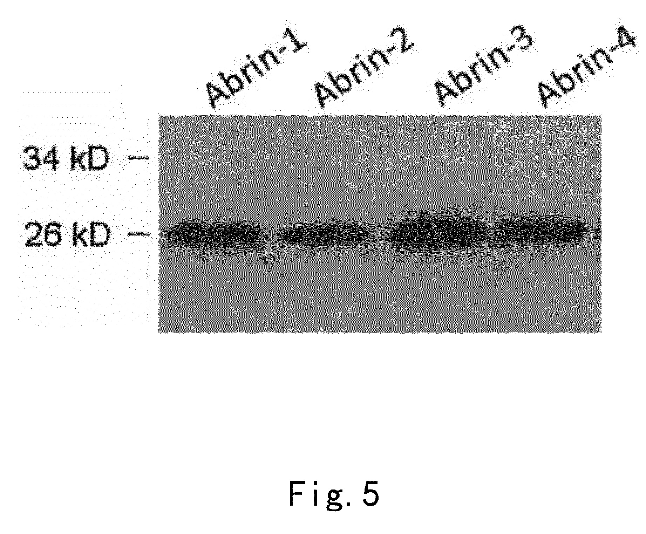

The disulfide bond between the chain A and chain B of abrin was reduced to open by treatment with 0.1M dithiothreitol (DTT). The epitope on abrin recognized by the antibodies was identified by Western blotting. As a result, it was found that a band at 26 kD but not a band at 34 kD appears for all of Abrin-1, Abrin-2, Abrin-3, and Abrin-4 (as shown in FIG.5), showing that all these four antibodies recognize the chain A of abrin (which has a molecular weight of about 30 kD).

### 6) Screening pairing antibodies by double-antibody sandwich ELISA method

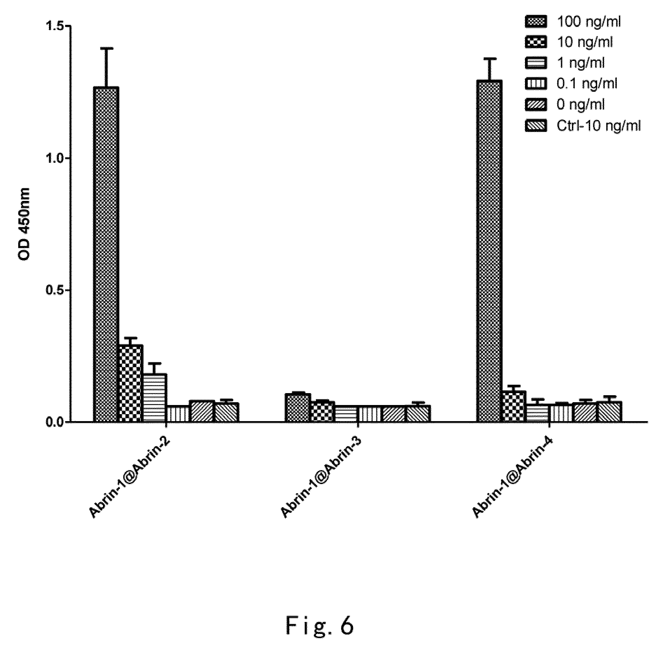

First, the antibody Abrin-1 was labeled with peroxidase HRP by glutaraldehyde two-step process or sodium periodate process. Then, the antibodies Abrin-2, Abrin-3, and Abrin-4 were diluted with 0.02 M PBS (pH 7.2) to 2 µg/ml, and then added at 50 µl/well to a 96-well plate, which was then coated at 4°C overnight, washed three times with PBST, blocked with 5% BSA-PBS for 1 h. 100, 10, 1, or 0.1 ng/ml abrin was added and 10 ng/ml ricin was added as a negative control (Ctrl), incubated at 37°C for 1 h, washed three times with PBST. The HRP-labeled Abrin-1 antibodies were added at a concentration of 1 µg/ml, incubated for 1 h, washed three times with PBST. Then, chromogenic substrate TMB (200 ng/ml TMB, 0.03% of H₂O₂, pH 4.5) was added at 50 µl/well for color development. The reaction was performed at 37°C for 15min. Thereafter, a 2M sulfuric acid solution was added at 50 µl/well to terminate the reaction. The results were obtained by using a microplate reader at 450 nm. From the results of ELISA (as shown in FIG.6), it can be seen that the best effect was achieved when pairing Abrin-1 with Abrin-2.

### 7) Preparation of Fe₃O₄ magnetic nanoparticles

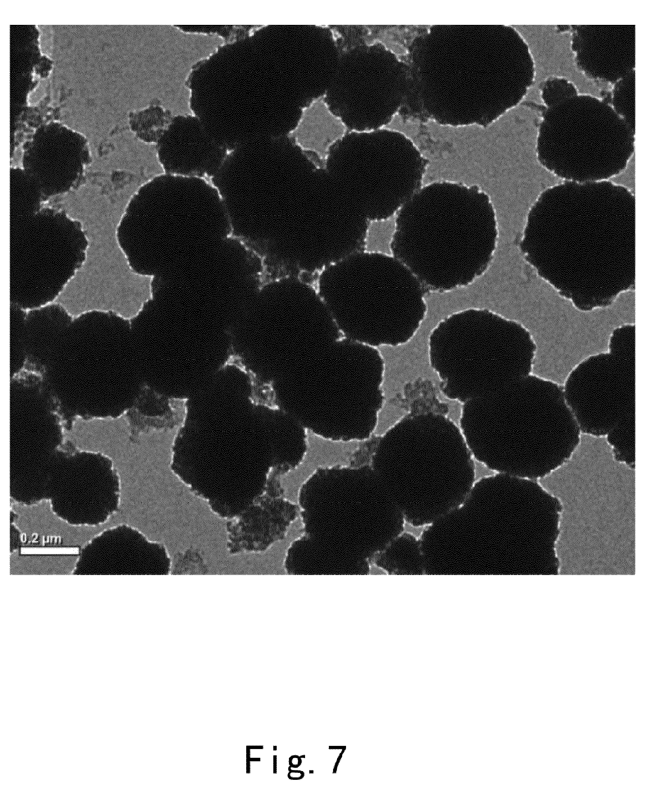

To a beaker pre-soaked in aqua regia, 0.3 g of CoCl₂-6H₂O, 0.675 g of FeCl₃-6H₂O, and 20 mL of ethylene glycol were added and completely dissolved by stirring. Then, 1.5 g anhydrous NaAc and 0.15 g PAA were added and stirred for another 30min. The reaction was performed at 200°C for 14h in a reactor, which was then cooled and the solution therein was poured into a tube. Then, magnetic separation was performed and the resulting supernatant was removed. The residue was then washed with ethanol for 4 times with ultrasonic treatment, dried at 50-60°C. The magnetic particle MNPs prepared had a particle size of about 350 nm (as shown in FIG.7).

### 8) Preparation of magnetic particle probes

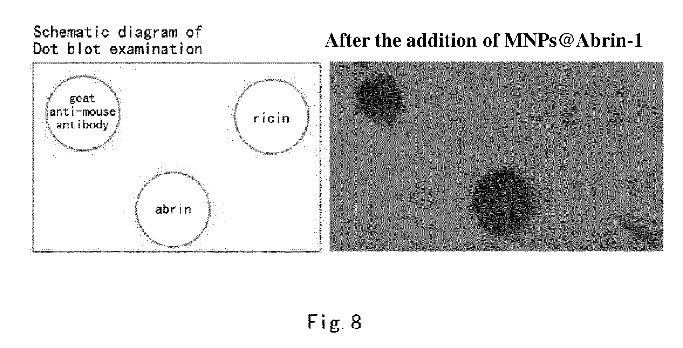

The carboxy groups on the surface of the magnetic particle were first activated with EDC-NHS (EDC: 1-(3-dimethylaminopropyl)-3-ethyl-carbodiimide hydrochloride; NHS: N-hydroxysuccinimide). Then, the abrin monoclonal antibodies Abrin-1 were coupled to 350 nm Fe₃O₄ magnetic nanoparticles to form MNPs@Abrin-1. Specifically, an appropriate amount of Fe₃O₄ magnetic nanoparticles were weighed. To the magnetic nanoparticles, 50 µl of NHS (50 mg/ml) and 50 µl of EDC (50 mg/ml) were added, incubated at room temperature for 30 min, and washed with DI water to remove excess NHS/EDC. 1 ml of sodium acetate solution (pH 6.0) and 100 µg of Abrin-1 antibody were added, mixed, incubated at 4°C for 2 hours, and washed with PBS. Then, the activated carboxyl groups were blocked with 50 mM Tris-Cl (pH 7.4), resuspended with PBS, and stored at 4°C. The effect of the coupling was determined by Dot blot method, in which goat anti-mouse antibody, abrin, and ricin (1 mg/ml, 1 µl, for each) were spotted on a nitrocellulose membrane and dried, and then a MNPs@Abrin-1 solution was added. The experimental results after the reaction showed that the magnetic particle probes only bind to the goat anti-mouse antibody and abrin but not to ricin, demonstrating excellent specificity of MNPs@Abrin-1 probe (as shown in FIG.8).

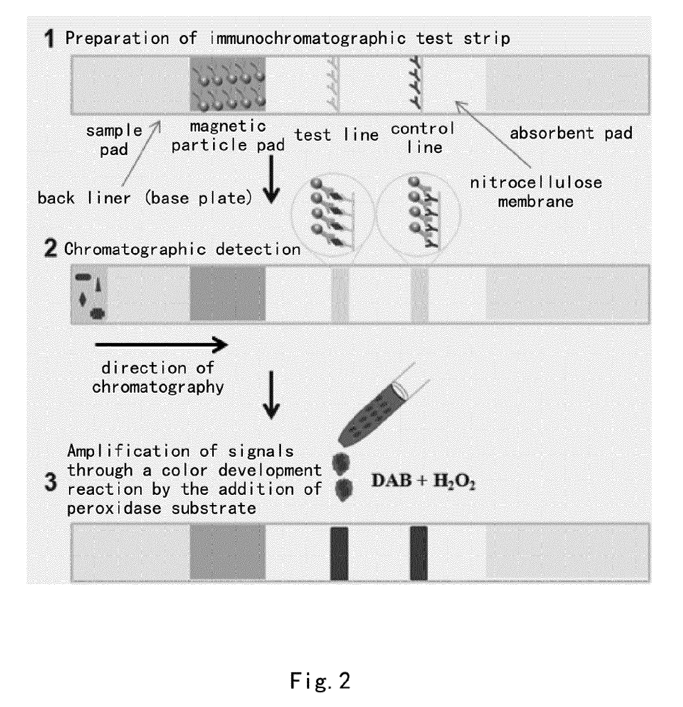

### 9) Assembling a test strip for nanozyme immunochromatographic method (as shown in FIG.2)

a. Preparation of a coating membrane: The abrin antibody Abrin-2 and a commercially available goat anti-mouse antibody (secondary antibody) were diluted with coating buffer (0.02 M phosphate buffer, pH 7.2) to 0.5 mg/ml and 1 mg/ml, respectively, and jet-printed uniformly on a nitrocellulose membrane of a width of 3.5 cm with an interval of 0.8 cm at 1 µl/cm by using a quantitative jet-printing apparatus to form a test line (T line) antibody band and a control line (C line) antibody band (the C line antibody band was behind the T line antibody band). After air drying at room temperature for 30 min, the membrane was soaked in a blocking buffer (0.02 M PBS containing 0.5% BSA, pH 7.2) for 10 min, and then dried at 25-35°C for 8 hours. Subsequently, a desiccant was added for storage.
b. Preparation of a magnetic particle probe pad: The magnetic particle probes as prepared in 8) were uniformly spray-coated on a glass fiber pad having a width of 0.8 cm at 50 µl/cm using the spray head of a sprayer, freeze-dried overnight, and then a desiccant was added for storage.
c. Treatment of sample pad: A sample pad (hydrophilic glass fiber) of a width of 1.8 cm was treated by soaking it in a sample pad processing solution (1-5% Casein, 0.1-1% PVA (polyvinyl alcohol), 0.01-0.2% Tween 20, 0.02 M PBS, pH 7.2) for 1 hour, and then removed and dried at 25-35°C for 8 hours.
d. Assembling and cutting of test strip: A 3.5 cm coating membrane, a 0.8 cm magnetic particle probe pad, a 1.8 cm sample pad, and a 2.5 cm water-absorbent pad were allowed to adhere onto a back liner (base plate) in such a manner that they were mutually staggered by 2mm and in the order as shown in Fig. 2. A layer of transparent plastic sealing film was placed as a cover. As such, a test paper board was assembled. The assembled test paper board was cut into a test strip of a width of 0.5 cm using a tape cutter. The cut test strip was then placed in the slot of a plastic base card. After covering with a lid, an upper plastic card and a lower plastic card were tightly pressed by using a card presser. Then, a desiccant was added for storage at room temperature. It would be appreciated that the sizes of the coating membrane, the magnetic particle probe pad, the sample pad, and the water-absorbent pad described above and the extent to which they are mutually staggered may be appropriately adjusted by the person skilled in the art as needed).

### 10) Loading and detection

50 µl of each of sample solutions containing abrin of various concentrations (100 ng/ml, 10 ng/ml, 1 ng/ml, and 0 ng/ml) was pipetted onto a sample pad on a detection card, and then 50 µl of chromatographic buffer (1% Tween 20, 0.5% Triton X-100, 1% NP-40, 0.05% NaN₃, 20 mM PBS, pH 7.2) was further added and reacted for 15 min. The abrin monoclonal antibodies Abrin-1 coupled to the magnetic particles bound to abrin in the sample solution to form a complex, which continued to move to the test line (T line) and bound to the antibodies Abrin-2 at the T line to generate aggregation of the magnetic particles. The magnetic particle-Abrin-1 antibody probes with no abrin bound would continue to move to the control line (C line) and bound to the secondary antibodies at the C line to generate aggregation of the magnetic particles.

### 11) Amplification of detection signal by nanozyme catalysis

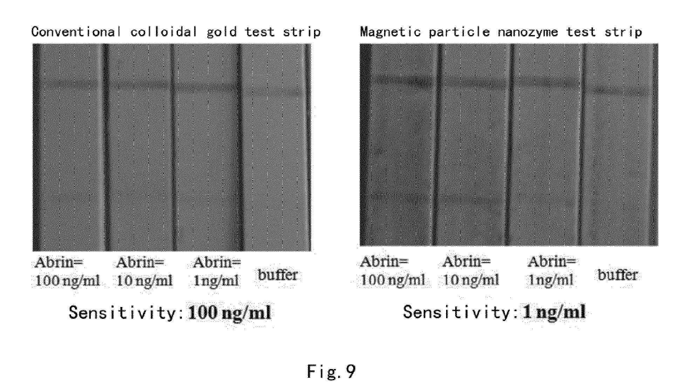

50 µl of a chromogenic buffer (530 mM H₂O₂; 816 mM 3,3'-diaminobenzidine (DAB) as the chromogenic substrate of peroxidase) was added at each of the test line (Abrin-2 antibody band) and the control line (goat anti-mouse antibody band) and reacted for 5 min. A large amount of brown insoluble precipitates were generated at the T line and the C line where the magnetic particles aggregated due to the nanozyme activity of the magnetic particles, so as to amplify detection signal. The sensitivity of the present detection method is 100 times of that of conventional detection method using colloidal gold test strips (as shown in FIG.9).

### Example 2: Detection of influenza virus by nanozyme immunochromatographic detection method

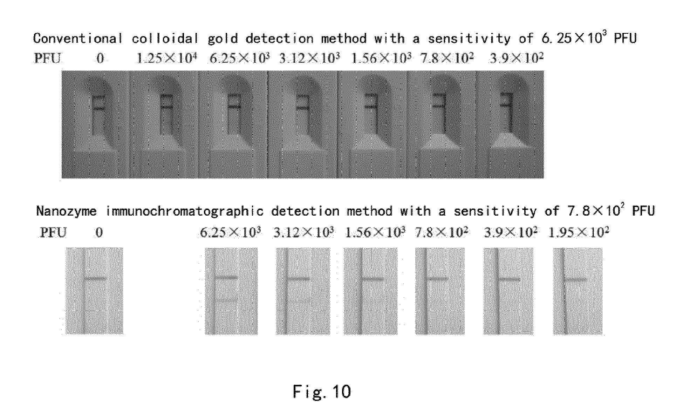

1) Source of the antibodies: commercially available, designated as FluA-1 and FluA-2 (manufacturer: Medix Biochemica, catalog Nos.: 100081 (FluA-1), 100083 (FluA-2)).
2) Preparation of Fe₃O₄ magnetic nanoparticles was the same as in Example 1.
3) Preparation of magnetic particle probes was the same as in Example 1 with the exception that the coupled antibody was influenza virus antibody FluA-1.
4) Assembling of test strip for nanozyme immunochromatographic method was the same as in Example 1, with the exception that the antibody at the test line (T line) was influenza virus antibody FluA-2.
5) Loading and detection
   50 µl of each of sample solutions containing various influenza virus concentrations (virus titer in PFU: 1.25×10⁴, 6.25×10³, 3.1×10³, 1.56×10³, 7.8×10², 3.9×10², and 1.95×10²) was pipetted on to a sample pad on a detection card, and 50 µl of chromatographic buffer (1% Tween 20, 0.5% Triton X-100, 1% NP-40, 0.05% NaN₃, 20 mM PBS, pH 7.2) was further added and reacted for 15 min. The influenza virus monoclonal antibodies FluA-1 coupled to the magnetic particles bound to the influenza viruses in the sample solutions to form a complex, which continued to move to the test line (T line) and bound to the influenza virus antibodies FluA-2 at the T line. The magnetic particle-FluA-1 antibody probes with no influenza virus bound would continue to move to the control line (C line) and bound to the secondary antibodies at the C line to generate aggregation of magnetic particles.
6) Amplification of detection signal by enzymatic catalysis
   50 µl of chromogenic buffer (530 mM H₂O₂; 3,3'-diaminobenzidine (DAB) as the chromogenic substrate of peroxidase) at a concentration of 816 mM) was added at each of the test line (FluA-2 antibody band) and the control line (goat anti-mouse antibody band), and reacted for 5 min. A large amount of brown insoluble precipitates were generated at the T line and the C line where the magnetic particles aggregated due to the peroxidase mimic activity of the magnetic particles, so as to amplify detection signal. The sensitivity of the present detection method is 8 times of that of conventional detection method using colloidal gold test strips (as shown in FIG. 10).

In summary, this invention provides a nanozyme immunochromatographic detection method. The present technique combines peroxidase catalytic activity of magnetic nanoparticles with magnetic separation property of magnetic nanoparticles. A large amount of brown precipitate is generated by adding a peroxide and a hydrogen-donor substrate such as o-phenylene diamine (DAB) and the like after chromatography by using enzymatic activity of the magnetic particles so as to amplify detection signal by 10-100 times, which enables the visualization of the detection results, and eliminates the dependency on instruments. This new technique is convenient and rapid and has a high sensitivity, and is quite suitable for on-site use. It is a new technique having novelty, inventiveness, and applicability.

Those described above are only examples of specific applications of the invention. It is to be understood that these examples are provided to illustrate this invention but are not intended to limit the scope of the invention.

## Claims

1. A nanozyme immunochromatographic detection method for detecting a substance to be tested in a liquid sample, comprising the steps of:
1) providing a detection probe, which is prepared by coupling a magnetic nanoparticle to a first molecule capable of specifically binding to the substance to be tested;
2) providing a capture probe, which is an immobilized second molecule capable of specifically binding to the substance to be tested;
3) bringing the liquid sample into contact with the detection probe;
4) bringing the liquid sample which has been in contact with the detection probe, into contact with the capture probe; and
5) adding a hydrogen-donor substrate and a peroxide to the capture probe which has been subjected to the step 4) so as to carry out a color development reaction.

2. The method according to claim 1, wherein the particle size of the magnetic nanoparticle is in a range of 10 nanometers to 500 nanometers.

3. The method according to claim 1, wherein the magnetic nanoparticle is Fe₃O₄ magnetic nanoparticle.

4. The method according to claim 1, wherein the substance to be tested is a protein, polypeptide, or nucleic acid.

5. The method according to claim 1, wherein the substance to be tested is protein, and the first molecule and the second molecule are specific antibodies, preferably monoclonal antibodies, against the protein.

6. The method according to claim 5, wherein the first molecule and the magnetic nanoparticle are coupled by EDC-NHS method.

7. The method according to claim 1, wherein the hydrogen-donor substrate includes tetramethyl benzidine (TMB), tetramethyl benzidine sulfate (TMBS), o-phenylene diamine (OPD), diaminobenzidine (DAB), diaminobenzidine tetrahydrochloride (DAB-4HCl), 5-amino salicylic acid (5-AS), o-tolidine (OT), or 2,2'-azino-bis(3-ethylbenzothiazoline-6-sulfonic acid) diammonium salt (ABTS).

8. The method according to claim 1, wherein the peroxide includes hydrogen peroxide and urea peroxide.

9. A nanozyme immunochromatographic detection apparatus for detecting a substance to be tested in a liquid sample, comprising the following provided on a base plate:
a sample pad, which is used for bearing the liquid sample and filtering impurities in the sample;
a magnetic nanoparticle pad, which comprises magnetic nanoparticles coupled to first molecules capable of specifically binding to the substance to be tested;
a test line, which comprises second molecules capable of specifically binding to the substance to be tested; and
an absorbent pad, which is generally made of a relatively thick filter paper or a similar water-absorbent material and is used for providing a driving force for chromatography,
wherein the particle size of the magnetic nanoparticle is preferably in a range of 10 nanometers to 500 nanometers,
wherein the magnetic nanoparticle is preferably Fe₃O₄ magnetic nanoparticle,
wherein the substance to be tested is preferably protein, polypeptide, or nucleic acid, and more preferably, the substance to be tested is protein, while the first molecule and the second molecule are specific antibodies, preferably monoclonal antibodies, against the protein,
wherein the first molecule and the magnetic nanoparticle are preferably coupled by EDC-NHS method,
wherein the hydrogen-donor substrate preferably includes tetramethyl benzidine (TMB), tetramethyl benzidine sulfate (TMBS), o-phenylene diamine (OPD), diaminobenzidine (DAB), diaminobenzidine tetrahydrochloride (DAB-4HCl), 5-amino salicylic acid (5-AS), o-tolidine (OT), or 2,2'-azino-bis(3-ethylbenzothiazoline-6-sulfonic acid) diammonium salt (ABTS), and
wherein the peroxide preferably includes hydrogen peroxide and urea peroxide.

10. The detection apparatus according to claim 9, further comprising, behind the test line, a control line at which third molecules capable of specifically binding to the first molecule are immobilized.
